# NEUE EUROPÄISCHE PATENTSCHRIFT

(11) **EP 1 218 455 B2**
(45) Veröffentlichungstag und Bekanntmachung des Hinweises auf die Entscheidung über den Einspruch: **09.10.2013**
(45) Hinweis auf die Patenterteilung: 21.04.2004
(21) Anmeldenummer: 00953169.0
(22) Anmeldetag: 16.08.2000
(51) Int. Cl.: C09C 1/00, C09D 17/00, C09D 5/36, C08K 9/02

(54) **PIGMENTMISCHUNG ENTHALTEND BiOCl-PIGMENTE**
PIGMENT MIXTURE THAT CONTAINS BiOC1 PIGMENTS
MELANGE DE PIGMENTS CONTENANT DES PIGMENTS DE BIOCL

(30) Priorität: 01.09.1999 DE 19941607
(43) Veröffentlichungstag der Anmeldung: 03.07.2002
(73) Patentinhaber: Merck Patent GmbH, 64293 Darmstadt (DE)
(72) Erfinder: ANSELMANN, Ralf, D-67305 Ramsau (DE); HILLGÄRTNER, Uta, D-64293 Darmstadt (DE); SCHOEN, Sabine, D-64287 Darmstadt (DE)
(86) Internationale Anmeldenummer: PCT/EP2000/007947
(87) Internationale Veröffentlichungsnummer: WO 2001/016235

(56) Entgegenhaltungen:
- EP-A- 0 960 911
- EP-A1- 0 960 911
- EP-A1- 1 013 724
- EP-A2- 0 498 686
- DE-A- 4 432 225
- DE-A1- 4 432 225
- DE-A1- 19 941 607
- US-A- 2 974 053
- US-A- 2 995 459
- US-A- 4 076 551
- Product Sheet Biju(r)BVW Engelhard Corporation
- Product Sheet Biju(r)Ultra HDF-20 Engelhard Corporation
- L.M. GREENSTEIN: '"Nacreous (Pearlescent) Pigments and Interference Pigments"' PIGMENT HANDBOOK Bd. 1, Seiten 829 - 858, XPSECOND ED.
- DAVID J.,ENGLER ET AL: 'Paint/Coatings Dictionary', 1978, FEDERATION OF SOCIETIES FOR COATINGS TECHNOLOGY
- ERNEST W., FLICK: 'Cosmetic and Toiletry Formulations, 2nd Edition', Bd. 3, 1995, NOYES PUBLICATIONS, NEW JERSEY
- Product Sheet Biju(r)Ultra UXD, BASF, Revision 3, 1995
- Product Sheet Timica Extra Large Sparkle Product No. 110 S, Mearl, Oktober 1981/March 1987
- BALSAM ET AL.: 'Cosmetics Science and Technology, 2nd Edition, chapter 29', Bd. 2, 1972, WILEY & SONS, INC., NEW YORK Seite 572
- Product Sheet Suspending Lacquer SLF-2, Mearl, November 1985
- E.W., FLICK: 'Cosmetics Additives: An Industrial Guide', 1991, NOYES PUBLICATIONS, NEW JERSEY Seite 812
- M.S. BALSAM ET AL: 'Cosmetics Science and Technology , 2nd ed., full version of chapter 29, previously partially submitted as D8c', Bd. 2, 1972, WILEY-INTERSCIENCE, NEW YORK Seiten 521 - 536
- Mearle Corp. technical information sheet for Biju(r)BVW dated 18 February 1993
- JOHANNE M., NIKITAKIS ET AL.: 'CTFA International Cosmetic Ingredient Dictionary, 4th Edition', 1991, THE COSMETIC, TOILETRY, AND FRAGRANCE ASSOCIATION, WASHINGTON Seite 688

## Beschreibung

Die vorliegende Erfindung betrifft die Verwendung von Pigmentmischungen in Dispersion, bestehend aus mindestens zwei Komponenten, wobei Komponente A BiOCI-Pigmente als Dispersion und Komponente B Perlglanzpigmente, plättchenförmige, nadelförmige oder sphärische Farbmittel und/oder Füllstoffe sind, in kosmetischen Formulierungen.

Da BiOCl-Pigmente einen hohen Brechungsindex (n = 2,15) und perlmuttartigen oder metallischen Silberglanz aufweisen, werden sie in Farben, Lacken, Kunststoffen und insbesondere in kosmetischen Produkten eingesetzt. Außer dem metallischen Silberglanz bzw. Perlglanz erwartet der Verbraucher von dekorativen Produkten immer mehr Funktionalität und stellt damit immer höhere Ansprüche an das Erscheinungsbild. Die aus der DE-PS 10 03 377, U.S. 2,975,053, DE 24 11 966, EP 0 496 686 B1 und DE 43 05 280 A1 bekannten BiOCl-Pigmente haben den Nachteil, dass sie entweder kein variables Deckvermögen, einen zu geringen metallischen Glanz, keine Absorptionsfarbe, keine Interferenzfarbe, und/oder keinen "Light-Diffusing-Effekt" aufweisen.

Aufgabe der vorliegenden Erfindung war es eine Pigmentmischung mit BiOCl-Pigmenten bereitzustellen, die sich durch ein hohes variables Deckvermögen bzw. variable Transparenz und/oder einen erhöhten metallischen Glanz auszeichnet, sich gut in das jeweilige Anwendungs-System einarbeiten lässt und dort stabil ist, und bei der eine optisch sichtbare Trennung BiOCI/Farbmittel bzw. BiOCl/Füllstoff im System weitgehend ausgeschlossen ist. Weiterhin sollte in kosmetischen Mitteln der Abrieb, Auftrag sowie das Hautgefühl gegenüber den Produkten im Markt verbessert werden.

Überraschenderweise wurde nun eine Pigmentmischung gefunden, die keine der oben angegebenen Nachteile aufweist. Das Pigmentgemisch besteht aus mindestens zwei Komponenten, wobei Komponente A beschichtete oder unbeschichtete BiOCl-Pigmente als Dispersion und Komponente B Perlglanzpigmente, insbesondere auf der Basis von Glimmer, SiO₂-, Glas-, Al₂O₃-, TiO₂-, Graphit- oder Polymerplättchen, plättchenförmige, nadelförmige oder sphärische Farbmittel und/oder Füllstoffe sind.

Durch die Zumischung der Komponente B zu den BiOCI-Pigmenten kann den Anwendungssystemen ein erhöhter metallischer Glanz verliehen werden, der Farbeffekt wird verstärkt und neuartige Farbeffekte werden erzielt. Gleichzeitig zeichnet sich das Pigmentgemisch durch sein variables, d.h. steuerbares, Deckvermögen von fast unsichtbar bis stark deckend aus. Darüber hinaus wird die Funktionalität des Endproduktes verbessert. Formulierungen enthaltend das Pigmentgemisch besitzen ein exzellentes Hautgefühl, eine hohe Hautaffinität, "Long Wear"-Eigenschaften, ein variables Deckvermögen, falls gewünscht einen metallischen Glanz, eine leichte Einarbeitbarkeit in das Endprodukt und eine vergleichsweise hohe Lichtstabilität.

Gegenstand der Erfindung ist somit die Verwendung von Pigmentmischungen gemäß der Ansprüche 1 und 2.

Die BiOCl-Pigmente können in jedem Verhältnis mit Komponente B gemischt werden. Vorzugsweise ist das Verhältnis von Komponente A zu Komponente B 1 : 90 bis 90 : 1, besonders bevorzugt 1 : 50 bis 50 : 1 und insbesondere 1 : 20 bis 20 : 1.

BiOCl-Pigmente sind kommerziell erhältlich und werden beispielsweise von der Fa. Merck KGaA, Deutschland unter den Markennamen Biron®, Bital®, Bicrona®, Mibiron®, Nailsyn® angeboten. Aufgrund der vielfältigen Produktionsmöglichkeiten sind BiOCl-Pigmente mit unterschiedlichen optischen Eigenschaften, von matt bis glänzend und von transparent bis hin zu deckend erhältlich. Die Größe der einzelnen Partikel liegt bei 1-100 µm, vorzugsweise bei 1-40 µm und insbesondere bei 2-35 µm. Die eingesetzten BiOCl-Pigmente können beschichtet oder unbeschichtet sein. Bei den beschichteten BiOCl-Pigmenten besteht die Beschichtung vorzugsweise aus Metalloxiden, wie z.B. TiO₂, TiO₂-Suboxiden, Fe₂O₃ und deren Gemischen oder aus organischen bzw. anorganischen Farbmitteln natürlichen und synthetischen Ursprungs. Bevorzugte Pigmentmischungen enthalten BiOCl-Pigmente, die mit Fe₂O₃, Carminrot, Berliner Blau oder Chromoxidgrün beschichtet sind.

Als Dispersion liegt das BiOCl vorzugsweise mit Nitrocelluloselacken oder Rizinusöl zunächst angeteigt vor und wird anschließend mit Komponente B gemischt. Das Pigmentgemisch als Dispersion zeichnet sich durch eine gute Dispergierbarkeit, pH-Stabilität, Hitzestabilität und Lagerstabilität im Endprodukt aus.

Neben den Anteigungen mit Rizinusöl und Nitrocelluloselacken sind ebenfalls Dispersionen in Wasser, polaren und unpolaren Ölen, Polyolen, hydrophilen und hydrophoben Lösemitteln, flüchtig oder nicht-flüchtig, geeignet.

Bevorzugte Pigmentgemische enthalten neben den BiOCl-Pigmenten (= Komponente A) als Farbmittel (= Komponente B) insbesondere ein oder mehrere Perlglanzpigmente. Als Perlglanzpigmente werden insbesondere Pigmente auf der Basis plättchenförmiger, transparenter oder semitransparenter Substrate aus z.B. Schichtsilikaten, wie etwa synthetischer oder natürlicher Glimmer, Talkum, Sericit, Kaolin, oder anderen silikatischen Materialien verwendet, die mit farbigen oder farblosen Metalloxiden wie z.B. TiO₂, Titansuboxide, Titanoxinitride, Pseudobrookit, Fe₂O₃, Fe₃O₄, FeOOH, SnO₂, Cr₂O₃, ZnO, CuO, NiO und anderen Metalloxiden allein oder in Mischung in einer einheitlichen Schicht oder in aufeinanderfolgenden Schichten beschichtet sind.

Perlglanzpigmente sind z.B. aus den deutschen Patenten und Patentanmeldungen 14 67 468, 19 59 998, 20 09 566, 22 14 454, 22 15 191, 22 44 298, 23 13 331, 25 22 572, 31 37 808, 31 37 809, 31 51 343, 31 51 354, 31 51 355, 32 11 602, 32 35 017, 38 42 330 und 44 45 394 bekannt und im Handel erhältlich, z.B. unter den Marken Colorona®, Timiron®, Dichrona®, Microna®, Soloron®, der Firma Merck KGaA, Darmstadt, Deutschland.

Besonders bevorzugte Pigmentpräparationen enthalten TiO₂-, Fe₂O₃-, TiO₂-Suboxide, TiO₂/Fe₂O₃-, Fe₃O₄-, FeOOH-, FeOOH/TiO₂-Glimmerpigmente. Weiterhin bevorzugt sind mit TiO₂ und/oder Fe₂O₃ beschichtete TiO₂-, Graphit-, Fe₂O₃-, SiO₂- oder Al₂O₃-Plättchen.

Als weitere plättchenförmige Pigmente kommen vor allem Perlglanzpigmente auf Basis von SiO₂-Plättchen, Al₂O₃-, Graphit-, Polymer-, TiO₂-Plättchen oder Glas-Plättchen, die mit einer oder mehreren Metalloxidschichten (ein, zwei, drei, fünf oder sieben) umhüllt sind, in Frage.

Weiterhin sind als Komponente B die beispielsweise aus den deutschen Offenlegungsschriften DE 196 18 563, DE 196 18 566, DE 196 18 569, DE 197 07 805, DE 197 07 806, DE 197 46 067 bekannten Mehrschichtpigmente geeignet. Diese basieren auf einer plättchenförmigen, transparenten, farbigen oder farblosen Matrix, bestehend aus Glimmer (synthetisch oder natürlich), SiO₂-Plättchen, Glas-Plättchen, Al₂O₃-Plättchen, TiO₂-Plättchen, Polymerplättchen, und besitzen in der Regel eine Dicke zwischen 0,3 und 5 µm, insbesondere zwischen 0,4 und 2,0 µm. Die Ausdehnung in den beiden anderen Dimensionen beträgt üblicherweise zwischen 1 und 250 µm, vorzugsweise zwischen 2 und 100 µm, und insbesondere zwischen 5 und 40 µm. Die Mehrschichtpigmente bestehen aus der Matrix (Substrat) beschichtet mit Metalloxiden (mindestens 2). Die Beschichtung der Substratplättchen, wie z. B. Glimmer, SiO₂-Plättchen, Glasplättchen, Al₂O₃-Plättchen, mit mehreren Schichten erfolgt so, dass ein Schichtaufbau bestehend aus alternierenden hoch- und niedrigbrechenden Schichten entsteht. Vorzugsweise enthalten die Mehrschichtpigmente 2, 3, 4, 5, 6 oder 7 Schichten, insbesondere 3, 4 oder 5 Schichten. Geeignete hochbrechende Metalloxide sind beispielsweise Titandioxid, Zirkonoxid, Zinkoxid, Eisenoxide, Eisen-Titan-Oxide (Eisentitanate) und/oder Chromoxid, insbesondere TiO₂ und/oder Fe₂O₃. Als niedrigbrechende Metalloxide kommen SiO₂ und Al₂O₃ zum Einsatz. Es kann hierfür jedoch auch MgF₂ oder ein organisches Polymer (z.B. Acrylat) eingesetzt werden. Die Beschichtung der Substratplättchen kann z.B. erfolgen wie in der WO 93/08237 (nasschemische Beschichtung) oder DE-OS-196 14 637 (CVD-Verfahren) beschrieben.

Bevorzugte Mehrschichtpigmente besitzen folgenden Aufbau:
Substrat + Fe₂O₃-Schicht + SiO₂-Schicht + Fe₂O₃-Schicht
Substrat + Fe₂O₃-Schicht + SiO₂-Schicht + TiO₂-Schicht
Substrat + TiO₂-Schicht + SiO₂-Schicht + Fe₂O₃-Schicht
Substrat + TiO₂-Schicht + SiO₂-Schicht + TiO₂/Fe₂O₃-Schicht
Substrat + TiO₂/Fe₂O₃-Schicht + SiO₂-Schicht + TiO₂/Fe₂O₃-Schicht
Substrat + TiO₂-Schicht + SiO₂-Schicht + Cr₂O₃-Schicht
Substrat + TiO₂-Schicht + SiO₂-Schicht + TiO₂-Schicht

Anstelle der äußeren Metalloxidschicht kann auch eine semitransparente Schicht eines Metalls verwendet werden. Geeignete Metalle dafür sind beispielsweise Cr, Ti, Mo, W, Al, Cu, Ag, Au oder Ni.

Zur Erzielung spezieller Farbeffekte können in die hoch- bzw. niedrigbrechenden Schichten zusätzlich noch feinteilige Partikel im Nanometergrößenbereich eingebracht werden. Als geeignet dafür erweisen sich beispielsweise feinteiliges TiO₂ oder feinteiliger Kohlenstoff (Ruß) mit Teilchengrößen im Bereich von 10-250 nm. Durch die lichtstreuenden Eigenschaften derartiger Partikel kann gezielt auf Glanz und Deckvermögen Einfluss genommen werden.

Die Perlglanz- oder Mehrschichtpigmente gemäß Komponente B können auch zur Verbesserung der Licht-, Wetter- und chemischen Stabilität oder zur Erhöhung der Kompatibilität in unterschiedlichen Medien noch mit einer Schutzschicht versehen sein. Als Nachbeschichtungen bzw. Nachbehandlungen kommen beispielsweise die in den DE 22 15 191, DE 31 51 354, DE 32 35 017 oder DE 33 34 598 beschriebenen Verfahren in Frage. Die zusätzlich aufgebrachten Stoffe machen nur etwa 0,1 bis 5 Gew.%, vorzugsweise 0,5 bis 3,0 Gew.%, des Mehrschichtpigments aus.

Als Komponente B für die Pigmentmischung sind alle dem Fachmann bekannten plättchenförmigen, nadelförmigen und sphärischen Farbmittel oder Füllstoffe geeignet, die eine Partikelgröße von 0,001 bis 20 µm, vorzugsweise 0,01 bis 5 µm, aufweisen. Bevorzugt enthalten die Pigmentgemische als Farbmittel Absorptionspigmente und als Füllstoffe plättchenförmige oder sphärische Pulver.

Insbesondere geeignet sind plättchenförmige Substrate, die auf Basis von Glimmer mit einem organischen und/oder anorganischen Farbstoff synthetischen oder natürlichen Ursprungs, beschichtet sind.

Zu den sphärischen kugelförmigen Farbmitteln zählen insbesondere TiO₂, BaSO₄, eingefärbtes oder beschichtetes SiO₂, CaSO₄, Eisenoxide, Chromoxide, Ruß, organische und anorganische Farbpigmente synthetischen oder natürlichen Ursprungs, wie z.B. Carmin, Berliner Blau, Anthrachinon-Pigmente, Chinacridon-Pigmente, Diketopyrrolopyrrol-Pigmente, Phthalocyanin-Pigmente, Azopigmente, Isoindolin-Pigmente. Von den sphärischen Farbmitteln ist das SiO₂ bevorzugt. Die SiO₂-Kugeln können beschichtet oder unbeschichtet sein. Vorzugsweise sind die SiO₂-Kugeln mit TiO₂, Fe₂O₃, FeOOH, Fe₃O₄, Berliner Blau oder Chromoxidgrün mit ein oder mehreren Schichten belegt. Vorzugsweise weisen die kugelförmigen Partikel, z.B. aus SiO₂, eine TiO₂/Fe₂O₃, SiO₂, TiO₂/Fe₂O₃-Beschichtung oder eine TiO₂, SiO₂, TiO₂-Beschichtung auf.

Insbesondere bevorzugt sind bei den mehrfach beschichteten SiO₂-Kugeln solche, die mit 3,5 oder 7 Schichten alternierend mit Metalloxiden unterschiedlicher Brechungsindices belegt sind. Kugelförmige SiO₂-Partikel mit einer Teilchengröße von 1 bis 30 µm sind im Handel erhältlich, z.B. unter den Marken Ronasphere®, Micronasphere® von der Fa. Merck KGaA.

Bei den nadelförmigen Pigmenten handelt es sich vorzugsweise um ZnO, Fe₂O₃, eingefärbte Glasfasern, α-FeOOH, organische Farbpigmente, wie z.B. Azopigmente, β-Phthalocyanin Cl Blue 15,3, Cromophtalgelb 8GN (Ciba-Geigy), Irgalith Blau PD56 (Ciba-Geigy), Azomethinkupferkomplex Cl Yellow 129, Irgazingelb 5GT (Ciba-Geigy). Insbesondere bevorzugt wird nadelförmiges Fe₂O₃ eingesetzt.

Empfehlenswert ist häufig die Zugabe eines UV-Stabilisators zu dem Pigmentgemisch in Mengen von 0,01-10 Gew.%, vorzugsweise von 0,01-5 Gew.% und insbesondere von 0,01-3 Gew.% bezogen auf den BiOCl-Gehalt. Besonders geeignete UV-Stabilisatoren sind solche, die im Handel unter dem Namen Eusolex® (Fa. Merck KGaA) erhältlich sind, wie z. B. Eusolex® 4360, ein 2-Hydroxy-4-methoxy-benzophenon. Weiterhin geeignet sind auch organische und anorganische Lichtschutzfilter als Pulver oder in Dispersion, z.B. auf Basis von mikronisiertem TiO₂ ,wie z. B. Eusolex® T-2000 oder Eusolex® T-Aqua.

Die Pigmentmischung ist einfach und leicht zu handhaben. Die Pigmentmischung kann durch einfaches Einrühren als Dispersion in das Anwendungssystem eingearbeitet werden. Ein aufwendiges Mahlen und Dispergieren der Pigmente ist nicht erforderlich.

Die Pigmentmischung kann zur Pigmentierung von kosmetischen Formulierungen, wie z.B. Lippenstiften, Nagellacken, kosmetische Stifte, Preßpuder, Make-ups, Shampoos, lose Puder und Gele, verwendet werden.

Die Konzentration der Pigmentmischung im zu pigmentierenden Anwendungssystem liegt in der Regel zwischen 0,1 und 70 Gew.%, vorzugsweise zwischen 0,1 und 50 Gew.% und insbesondere zwischen 1,0 und 20 Gew.%, bezogen auf den Gesamtfestkörpergehalt des Systems. Sie ist in der Regel abhängig vom konkreten Anwendungsfall.

Das Pigmentgemisch kann auch vorteilhaft in der dekorativen und pflegenden Kosmetik eingesetzt werden. Die hochglänzenden BiOCl-Dispersionen in Kombination mit Komponente B werden vorzugsweise in Pasten eingesetzt, insbesondere in Lippenstiften und Nagellacken. In der dekorativen Kosmetik ermöglichen die Pigmentmischungen ein besonders gleichmäßiges Auftragen des Puders auf der Haut und führen zu einer Verbesserung des Hautgefühls und zu einem Light Diffusing Wrinkle Hiding-Effekt. Außerdem wird die Hauthaftung verbessert und die Rissbildung während der Verpressung verhindert. Weiterhin zeigt das Pigmentgemisch in der kosmetischen Formulierung eine Verbesserung des Abriebs bzw. Auftrags und der Verteilung, ein variables Deckvermögen von transparent bis deckend und/oder einen Glanz von matt bis glänzend.

Die Einsatzkonzentration und das Mischungsverhältnis von BiOCl-Pigmenten mit Komponente B, insbesondere organischen und anorganischen Farbpigmenten und Farbstoffen, natürlichen oder synthetischen Ursprungs, wie z.B. Chromoxid, Ultramarin, beschichteten oder unbeschichteten sphärischen SiO₂- oder TiO₂-Pigmenten, wie z. B. aus der DE-OS-198 42 134 bekannt, sind abhängig vom Anwendungsmedium und dem Effekt, der erzielt werden soll. Die Mischung aus BiOCI-Pigmenten als Dispersion mit anderen Pigmenten oder Farbstoffen kann in allen Verhältnissen erfolgen, vorzugsweise beträgt das Verhältnis 1 : 10 bis 10 : 1. Die Einsatzkonzentration reicht von 1 Gew.% im Nagellack bis zu 70 Gew. % im Presspuder. Bei einer Mischung von BiOCl-Pigmenten mit sphärischen Füllstoffen, z. B. SiO₂, kann die Konzentration bei 0,01-70 Gew.% in der Formulierung liegen. Die kosmetischen Produkte, wie z.B. Nagellacke, Lippenstifte, Presspuder, Shampoos, lose Puder und Gele, zeichnen sich durch besonders interessante Glanz- und/oder Farbeffekte aus. In Lippenstiften und Nagellacken kommt die Pigmentmischung insbesondere als Dispersion zum Einsatz. Für den Einsatz in Lippenstiften werden vorzugsweise 70%ige Pigmentgemisch-Anteigungen verwendet. Nagellacke enthalten vorzugsweise thixotrope, toluolfreie formaldehydfreie Nitrocellulose-Anteigungen des Pigmentgemisches. Die Nitrocellulose-Anteigungen enthalten in der Regel 40-75 Gew.% Nitrocellulose und 25-60 Gew.% Pigmentgemisch. Der metallische Silberglanz im Nagellack kann gegenüber herkömmlichen Nagellacken mit Hilfe der Pigmentgemische deutlich gesteigert werden. Weiterhin kann das Pigmentgemisch in Badezusätzen und Zahnpasten eingesetzt werden.

Die nachfolgenden Beispiele sollen die Erfindung erläutern, ohne sie jedoch zu begrenzen.

### Beispiele:

### Beispiel 1

### Long-Lasting Lippenstift mit 10 % Perlglanzpigment und 5 % Biron® MTU

| Phase | Inhaltsstoff | Zusammensetzung | Hersteller | % |
|---|---|---|---|---|
| A | Colorona® Bright Gold | TiO₂/Fe₂O₃-Glimmerpigment der Teilchengröße 10-60 µm | (1) | 5,00 |
| | Colorona® Bordeaux⁻ | Fe₂O₃-Glimmerpigment der Teilchengröße 10-60 µm | (1) | 5,00 |
| | Biron® MTU | BiOCI-Pigment der Teilchengröße 15 µm | (1) | 5,00 |
| | | | | |
| B | Covalip LL 48 | Ozokerite, Candellila Cera, Isostearyl Alcohol, Isopropyl Palmitate, Myristyl Lactate, Cera Alba, Copernicia Cerifera, Quaternium-18 Hectorite, Propylene, Carbonate, Ethylene/VA Copolymer, Propylparaben | (2) | 44,00 |
| | Dow Corning 556 | Phenyl Trimethicone | (3) | 2,70 |
| | (-)-α-Bisabolol | Bisabolol | (1) | 0,30 |
| | | | | |
| C | Dow Corning 345 | Cyclomethicone | (3) | 28,00 |
| | Pigment Rouge Covasil W 3801 C, (10 % in DC 345) | Fe₂O₃, Cyclomethicone | (2) | 10,00 |

### Herstellung:

Alle Bestandteile der Phase B zusammen aufschmelzen, rühren bis alles geschmolzen ist. Die Perlglanzpigmente und die BiOCI-Pigmente der Phase A einrühren. Phase C langsam zugeben, unter Rühren die Masse auf ca. 60 °C abkühlen, in zwei Etappen in Slimstick-Container gießen. Rezeptur muss in einer Ex-geschützten Apparatur hergestellt werden.

### Bezugsquellen:

(1) Merck KGaA
(2) Les Colorants Wackherr
(3) Dow Corning

### Beispiel 2

### Rouge

| Phase | Inhaltsstoff | Zusammensetzung | Hersteller | % |
|---|---|---|---|---|
| A | Microna® Matte Red | Fe₂O₃-Glimmerpigment der Teilchengröße < 15 µm | (1) | 21,50 |
| | Microna® Matte White | TiO₂/ZnO-Glimmerpigment der Teilchengröße < 15 µm | (1) | 7,00 |
| | Microna® Matte Orange | Fe₂O₃-Glimmerpigment der Teilchengröße < 15 µm | (1) | 0,50 |
| | Microna® Matte Yellow | FeOOH-Glimmerpigment der Teilchengröße < 15 µm | (1) | 0,50 |
| | Microna® Matte Black | Fe₃O₄-Glimmerpigment der Teilchengröße < 15 µm | (1) | 0,50 |
| | Biron® ESQ | BiOCI-Pigment | (1) | 5,00 |
| | Micronasphere® M | Mica/SiO₂ | (1) | 5,00 |
| | Talkum | Talc | (1) | 18,00 |
| | Weißer Ton | Kaolin | (1) | 25,00 |
| | Reisstärke | Oryza Sativa (Rice Starch) | (1) | 5,00 |
| | Magnesiumstearat | Magnesium Stearate | (1) | 2,00 |
| | | | | |
| B | Isopropylmyristat | Isopropyl Myristate | (1) | 8,00 |
| | Dow Corning Q2-1403 fluid | Dimethicone, Dimethiconol | (2) | 1,00 |
| | Dow Corning 200 (350 cs) fluid | Dimethicone | (2) | 1,00 |

### Herstellung:

Bestandteile der Phase A zusammengeben, vormischen und sieben (100 µm). Anschließend tropfenweise den Binder einrühren. Die Puder werden bei 40-50 bar gepresst.

### Bezugsquellen:

(1) Merck KGaA
(2) Dow Corning

### Beispiel 3

### Lippenstift mit 5 % Perlglanzpigmenten und 14,30 % Biron Silver CO

| Phase | Inhaltsstoff | Zusammensetzung | Hersteller | % |
|---|---|---|---|---|
| A | Biron® Silver CO | BiOCl-Pigment in Rizinusöl | (1) | 14,30 |
| | Colorona® Imperial Red | TiO₂/Glimmerpigment + Cl 73360 (D&C Red # 30) der Teilchengröße 10-60 µm | (1) | 5,00 |
| | | | | |
| B | Bienenwachs | Cera Alba (Beeswax) | (1) | 8,75 |
| | Paracera C 44 | Ceresin, Copernicia Cerifera (Carnauba Wax) | (2) | 5,25 |
| | Adeps Lanae SP | Lanolin | (3) | 3,50 |
| | Isopropylmyristat | Isopropyl Myristate | (1) | 5,60 |
| | Paraffin dickflüssig | Paraffinum Liquidum (Mineral Oil) | (1) | 2,10 |
| | Oxynex® K flüssig | PEG-8, Tocopherol, Ascorbyl Palmitate, Ascorbic Acid, Citric Acid | (1) | 0,05 |
| | Konservierung | | | q.s. |
| | Rizinusöl | Ricinus Communis (Castor Oil) | (3) | 54,45 |
| | Eusolex® 4360 | Benzophenone-3 | (1) | 1,00 |
| | | | | |
| C | Parfümöl | Parfum | | q.s. |

### Herstellung:

Die Bestandteile der Phase B werden auf 75 °C erhitzt und aufgeschmolzen. Die Pigmente der Phase A werden zugegeben und alles gut durchrührt. Die Lippenstiftmasse wird dann in der auf 65 °C temperierten Gießapparatur 15 Minuten gerührt und parfümiert. Die homogene Schmelze wird in die auf 55 °C vorgewärmten Gießformen gegossen. Anschließend kühlt man die Formen ab und entfernt die Gießlinge kalt. Nach Erwärmen der Lippenstifte auf Raumtemperatur werden die Lippenstifte kurz abgeflammt.

### Bezugsquellen:

(1) Merck KGaA
(2) Paramelt
(3) Henry Lamotte
(4) Haarmann & Reimer

### Beispiel 4

### Lidschatten

| Phase | Inhaltsstoff | Zusammensetzung | Hersteller | % |
|---|---|---|---|---|
| A | Biron® B 50 | BiOCl-Pigment der Teilchengröße 2-35 µm | (1) | 3,00 |
| | Microna® Matte Black | Fe₃O₄/Glimmerpigment der Teilchengröße <15 µm | (1) | 0,50 |
| | Ronasphere® | Silica | (1) | 5,00 |
| | Magnesiumstearat | Magnesium Stearate | (1) | 2,50 |
| | Weißer Ton | Kaolin | (1) | 5,00 |
| | Hubersorb 600 | Calcium Silicate | (2) | 0,50 |
| | Talkum | Talc | (1) | 15,80 |
| | | | | |
| B | Colorona® Magenta | TiO₂/Glimmerpigment + Cl 75470 (Carmine) der Teilchengröße 10-60 µm | (1) | 55,00 |
| | | | | |
| C | Amerchol L-101 | Mineral Oil, Lanolin Alcohol | (3) | 10,70 |
| | Super Hartolan | Lanolin Alcohol | (4) | 1,00 |
| | Ewalin 1751 | Petrolatum | (5) | 1,00 |
| | Konservierung | | | q.s. |
| | Parfümöl | | | q.s. |

### Herstellung:

Bestandteile der Phase A zusammen geben und durch 63 µm sieben. Anschließend Phase B zugeben und die Pudermischung unter kräftigem Rühren tropfenweise mit der geschmolzenen Phase C versetzen. Die Puder werden bei 40-50 bar gepresst.

### Bezugsquellen:

(1) Merck KGaA
(2) Huber
(3) Amerchol
(4) Croda
(5) H. E. Wagner

### Beispiel 5

### Lippenpuder mit 30 % Perlglanzpigment und 10 % Biron® Fines

| Phase | Inhaltsstoff | Zusammensetzung | Hersteller | % |
|---|---|---|---|---|
| A | Colorona® Bordeaux | Fe₂O₃-Glimmerpigment | (1) | 30,0 |
| | Biron® Fines | BiOCl-Pigment der Teilchengröße 2-20 µm | (1) | 10,0 |
| | Talkum | Talc | (1) | 30,0 |
| | Ronasphere® LDP | Silica mit TiO₂/ Fe₂O₃-Beschichtung | | 10,0 |
| | Magnesiumstearat | Magnesium Stearate | (1) | 5,0 |
| | | | | |
| B | Isopropylstearat | Isopropyl Stearate | (2) | 11,2 |
| | Dow Corning 1403 | Dimethicone, | (3) | 3,8 |
| | fluid | Dimethiconol | | |
| | Parfümöl | | | q.s. |
| | Konservierung | Propylparaben | | q.s. |

### Herstellung:

Bestandteile der Phase A zusammen geben und vormischen. Anschließend die Pudermischung unter Rühren tropfenweise mit der geschmolzenen Phase B versetzen. Die Puder werden bei 40-50 bar gepresst.

### Bezugsquellen:

(1) Merck KGaA
(2) Henkel KGaA
(3) Dow Corning

### Beispiel 6

### Creamy Eyeshadow

| Phase | Inhaltsstoff | Zusammensetzung | Hersteller | % |
|---|---|---|---|---|
| A | Colorona® Dark Blue | TiO₂-Glimmerpigment mit Berliner Blau der Teilchengröße 10-60 µm | (1) | 10,00 |
| | Timiron® Supersheen MP-1001 | TiO₂-Glimmerpigment der Teilchengröße 5-25 µm | (1) | 10,00 |
| | Biron® LF 2000 | BiOCI-Pigmente der Teilchengröße <20 µm | (1) | 10,00 |
| | Talkum | Talc | (1) | 10,00 |
| | | | | |
| B | Crodamol PMP | PEG-2 Myristyl Ether Propionate | (2) | 32,90 |
| | Syncrowachs HGLC | C18-36 Acid Triglyceride | (2) | 10,00 |
| | Syncrowachs HRC | Tribehenin | (2) | 3,00 |
| | Miglyol 812 Neutralöl | Caprylic /Capric Triglyceride | (3) | 9,00 |
| | Stearinsäure | Stearic Acid | (1) | 3,00 |
| | Antaron V-216 | PVP/Hexadecene Copolymer | (4) | 2,00 |
| | Oxynex® K flüssig | Tocopherol, Ascorbyl Palmitate, Ascorbic Acid, Citric Acid, PEG-8 | (1) | 0,10 |
| | Konservierung | Propylparaben | | q.s. |

### Herstellung:

Phase B auf ca. 80 °C erhitzten bis alles geschmolzen ist und auf 65 °C abkühlen. Unter Rühren werden nun die Inhaltsstoffe der Phase A zugegeben und der fertige Lidschatten noch flüssig abgefüllt.

### Bezugsquellen:

(1) Merck KGaA
(2) Croda
(3) Hüls AG
(4) ISP Europe

### Beispiel 7

### Nagellack

| Inhaltsstoff | Zusammensetzung | Hersteller | % |
|---|---|---|---|
| Nagellackbase 2702 | Nitrocellulose Base | (2) | 89,75 |
| Timiron® Splendid Green | TiO₂/SiO₂-Glimmerpigment der Teilchengröße 10-60 µm | (1) | 2,00 |
| Nailsyn® Sterling 60 | BiOCl in Nitrocellulose | (1) | 5,00 |
| Yellow HO 203 | FD&C Yellow No. 5 in Nitrocellulose Basis | (2) | 0,90 |
| Blue HO 208 | Berliner Blau in Nitrocellulose | (2) | 0,35 |
| White HO 1270 | TiO₂ in Nitrocellulose | (2) | 2,00 |

### Herstellung:

Die BiOCl-Dispersion und die Pigmente werden nacheinander unter Rühren der Nagellackbase zugegeben. Anschließend wird bei 1000 Upm 10 min weitergerührt.

### Bezugsquellen:

(1) Merck KGaA
(2) International Lacquers

### Beispiel 8

### Lidschatten

| Phase | Inhaltsstoff | Zusammensetzung | Hersteller | % |
|---|---|---|---|---|
| A | | mit Fe₂O₃ beschichtetes BiOCl-Pigment der Teilchengröße 2-35 µm | (1) | 3,50 |
| | Ronasphere® | Silica | (1) | 5,00 |
| | Magnesiumstearat | Magnesium Stearate | (1) | 2,50 |
| | Weißer Ton | Kaolin | (1) | 5,00 |
| | Hubersorb 600 | Calcium Silicate | (2) | 0,50 |
| | Talkum | Talc | (1) | 15,80 |
| B | Corona® Magenta | TiO₂/Glimmerpigment + Cl 75470 (Carmine) der Teilchengröße 10-60 µm | (1) | 55,00 |
| C | Amerchol L-101 | Mineral Oil, Lanolin, Alcohol | (3) | 10,70 |
| | Super Hartolan | Lanolin Alcohol | (4) | 1,00 |
| | Ewalin 1751 | Petrolatum | (5) | 1,00 |
| | Konservierung | | | q.s. |
| | Parfümöl | | | q.s. |

### Herstellung:

Bestandteile der Phase A zusammen geben und durch 63 µm sieben. Anschließend Phase B zugeben und die Pudermischung unter kräftigem Rühren tropfenweise mit der geschmolzenen Phase C versetzen. Die Puder werden bei 40-50 bar gepresst.

### Bezugsquellen:

(1) Merck KGaA
(2) Huber
(3) Amerchol
(4) Croda
(5) H.E. Wagner

### Beispiel 9

### Lippenpuder mit 30 % Perlglanzpigment und 10 % Biron® Fines

| Phase | Inhaltsstoff | Zusammensetzung | Hersteller | % |
|---|---|---|---|---|
| A | Bicrona® Carmine | mit Carminrot beschichtetes BiOCl-Pigment der Teilchengröße 2-20 µm | (1) | 40,0 |
| | Talkum | Talc | (1) | 30,0 |
| | Ronasphere® LDP | Silica mit TiO₂/Fe₂O₃-Beschichtung | | 10,0 |
| | Magnesiumstearat | Magnesium Stearate | (1) | 5,0 |
| B | Isopropylstearat | Isopropyl Stearate | (2) | 11,2 |
| | Dow Corning 1403 | Dimethicone, Dimethiconol | (3) | 3,8 |
| | fluid | | | |
| | Parfümöl | | | q.s. |
| | Konservierung | Propylparaben | | q.s. |

### Herstellung:

Bestandteile der Phase A zusammen geben und vormischen. Anschliessend die Pudermischung unter Rühren tropfenweise mit der geschmolzenen Phase B versetzen. Die Puder werden bei 40-50 bar gepresst.

### Bezugsquellen:

(1) Merck KGaA
(2) Henkel KGaA
(3) Dow Corning

### Beispiel 10

### Kompaktpuder

| Phase | Inhaltsstoff | Zusammensetzung | Hersteller | % |
|---|---|---|---|---|
| A | Microna® Matte White | TiO₂/ZnO-Glimmerpigment der Teilchengröße 2-15 µm | (1) | 18,6 |
| | Microna® Matte Orange | Fe₂O₃-Glimmerpigment der Teilchengröße < 15 µm | (1) | 3,8 |
| | Microna® Matte Yellow | FeOOH-Glimmerpigment der Teilchengröße < 15 µm | (1) | 3,8 |
| | Microna® Matte Black | Fe₃O₄-Glimmerpigment der Teilchengröße < 15 µm | (1) | 3,8 |
| | Biron® ESQ | BiOCl-Pigment | (1) | 10,0 |
| | Satin Mica | Mica | (1) | ad |
| | | | | 100% |
| | Orgaso12002 | Nylon-12 | (2) | 6,0 |
| | Talkum | Talc | (1) | 10,0 |
| | Ronasphere® LDP | Silica mit TiO₂/Fe₂O₃-Beschichtung | (1) | 10,0 |
| B | Hest CSO | Cetearyl Octanoate | (3) | 1,0 |
| | Ivarlan 3350 | Isopropyl Lanolate | (4) | 1,5 |
| | Crodamol OP | Octylpalmitate | (5) | 3,0 |
| | Parfümöl | Parfum | | q.s. |
| | Konservierung | | | q.s. |

### Herstellung:

Bestandteile der Phase A zusammengeben, vormischen und sieben (100 µm). Anschließend tropfenweise den gelösten Binder der Phase B einrühren bis eine homogene Verteilung erreicht ist. Anschließend abfüllen und pressen.

### Bezugsquellen:

(1) Merck KgaA
(2) Elf Atochem
(3) Heterene
(4) Brooks
(5) Croda

### Beispiel 11

### Two Way Cake Make Up

| Phase | Inhaltsstoff | Zusammensetzung | Hersteller | % |
|---|---|---|---|---|
| A | Microna® Matte White | TiO₂/ZnO-Glimmerpigment der Teilchengröße 2-15 µm | (1) | 18,6 |
| | Microna® Matte Orange | Fe₂O₃-Glimmerpigment der Teilchengröße < 15 µm | (1) | 3,8 |
| | Microna® Matte Yellow | FeOOH-Glimmerpigment der Teilchengröße < 15 µm | (1) | 3,8 |
| | Microna® Matte Black | Fe₃O₄-Glimmerpigment der Teilchengröße < 15 µm | (1) | 3,8 |
| | Biron® MTU | BiOCI-Pigment | (1) | 10,0 |
| | Satin Mica | Mica | (1) | ad |
| | | | | 100% |
| | Orgasl 2002 | Nylon-12 | (2) | 6,0 |
| | Talkum | Talc | (1) | 20,0 |
| | Ronasphere® | Silica mit TiO₂/Fe₂O₃- | (1) | 10,0 |
| | LDP | Beschichtung | | |
| B | | Hydrogenated Castor Oil | (3) | 2,0 |
| | | Propylenglycol Caprilate | (4) | 5,0 |
| | | Sorbitan Oleate (P.OE)20 | (5) | 0,1 |
| | Crill 3 | Sorbitan Stearate | (5) | 0,1 |
| | Parfümöl | Parfum | | 0,5 |
| | Konservierung | | | 0,2 |

### Herstellung:

Bestandteile der Phase A zusammengeben, vormischen und sieben (100 µm). Bestandteile der Phase B mischen und erhitzen bis sich alles gelöst hat. Phase B zu Phase A geben, bis eine homogene Verteilung erfolgt ist und abfüllen.

### Bezugsquelle:

(1) Merck KgaA
(2) Elf Atochem
(3) Cognis
(4) Nikko
(5) Croda

## Patentansprüche

1. Verwendung von Pigmentmischungen in Dispersion in kosmetischen Formulierungen, **dadurch gekennzeichnet, dass** sie mindestens zwei Komponenten enthalten, wobei Komponente A unbeschichtete BiOCl-Pigmente sind, die als Dispersion in Rizinusöl, Nitrocelluloselack, Wasser, polaren und unpolaren Ölen, Polyolen, hydrophilen und hydrophoben Lösemitteln, flüchtig oder nicht-flüchtig, vorliegen und Komponente B Perlglanzpigmente
ausgewählt aus
- Perlglanzpigmenten auf Basis von Fe₂O₃-, Glas-, TiO₂-, SiO₂-, Al₂O₃-, Polymer- oder Graphitplättchen,
- Mehrschichtpigmenten,
- Fe₂O₃-, TiO₂-Suboxiden-, TiO₂/Fe₂O₃-, Fe₃O₄-, FeOOH-, FeOOH/TiO₂-Glimmerpigmenten
und/oder
plättchenförmige, nadelförmige oder sphärische Farbmittel und/oder Füllstoffe sind.

2. Verwendung von Pigmentmischungen in Dispersion in kosmetischen Formulierungen, **dadurch gekennzeichnet, dass** sie mindestens zwei Komponenten enthalten, wobei Komponente A beschichtete BiOCl-Pigmente sind, die als Dispersion in Rizinusöl, Nitrocelluloselack, polaren und unpolaren Ölen, Polyolen, hydrophilen und hydrophoben Lösemitteln, flüchtig oder nicht-flüchtig, vorliegen und Komponente B Perlglanzpigmente, plättchenförmige, nadelförmige oder sphärische Farbmittel und/oder Füllstoffe sind.

3. Verwendung nach Anspruch 2, **dadurch gekennzeichnet, dass** Komponente A BiOCl-Pigmente enthält, die mit Fe₂O₃, Carminrot, Berliner Blau oder Chromoxidgrün beschichtet sind.

4. Verwendung nach Anspruch 2 oder 3, **dadurch gekennzeichnet, dass** Komponente B ein Perlglanzpigment auf Basis von Glimmer-, Fe₂O₃-, Glas-, TiO₂-, SiO₂-, Al₂O₃-, Polymer- oder Graphitplättchen ist.

5. Verwendung nach einem der Ansprüche 1 bis 4, **dadurch gekennzeichnet, dass** das Perlglanzpigment ein Mehrschichtpigment ist.

6. Verwendung nach Anspruch 5, **dadurch gekennzeichnet, dass** Komponente B ein Mehrschichtpigment enthält, das eine Fe₂O₃, SiO₂, Fe₂O₃-Beschichtung, eine TiO₂/Fe₂O₃, SiO₂, TiO₂/Fe₂O₃-Beschichtung oder eine TiO₂, SiO₂, TiO₂-Beschichtung aufweist.

7. Verwendung nach einem der Ansprüche 1 bis 3, **dadurch gekennzeichnet, dass** Komponente B mit organischen und/oder anorganischen Farbstoffen synthetischen oder natürlichen Ursprungs beschichtete Glimmerplättchen, sphärisches SiO₂ beschichtet oder unbeschichtet, Ruß, organische Farbpigmente und/oder anorganische Farbpigmente enthält.

8. Verwendung nach einem der Ansprüche 1 bis 7, **dadurch gekennzeichnet, dass** Komponente A und Komponente B im Verhältnis 90 : 1 bis 1 : 90 gemischt sind.

9. Verwendung nach einem der Ansprüche 1 bis 8, **dadurch gekennzeichnet, dass** sie zusätzlich einen UV-Stabilisator enthält.

## Claims

1. Use of pigment mixtures in dispersion in cosmetic formulations, **characterised in that** they comprise at least two components, where component A comprises uncoated BiOCl pigments in the form of a dispersion in castor oil, nitrocellulose lacquer, water, polar and nonpolar oils, polyols, hydrophilic and hydrophobic solvents, volatile or non-volatile, and component B comprises pearlescent pigments selected from
- pearlescent pigments based on Fe₂O₃, glass, TiO₂, SiO₂, Al₂O₃, polymer or graphite flakes,
- multilayered pigments,
- Fe₂O₃-, TiO₂ suboxide-, TiO₂/Fe₂O₃-, Fe₃O₄-, FeOOH-, FeOOH/TiO₂-mica pigments
and/or
flake-form, needle-shaped or spherical colorants and/or fillers.

2. Use of pigment mixtures in dispersion in cosmetic formulations, **characterised in that** they comprise at least two components, where component A comprises coated BiOCl pigments in the form of a dispersion in castor oil, nitrocellulose lacquer, polar and nonpolar oils, polyols, hydrophilic and hydrophobic solvents, volatile or non-volatile, and component B comprises pearlescent pigments, flake-form, needle-shaped or spherical colorants and/or fillers.

3. Use according to Claim 2, **characterised in that** component A comprises BiOCl pigments which are coated with Fe₂O₃, Carmine Red, Berlin Blue or Chromium Oxide Green.

4. Use according to Claim 2 or 3, **characterised in that** component B is a pearlescent pigment based on mica flakes, Fe₂O₃ flakes, glass flakes, TiO₂ flakes, SiO₂ flakes, Al₂O₃ flakes, polymer flakes or graphite flakes.

5. Use according to one of Claims 1 to 4, **characterised in that** the pearlescent pigment is a multilayered pigment.

6. Use according to Claim 5, **characterised in that** component B comprises a multilayered pigment which has an Fe₂O₃, SiO₂, Fe₂O₃ coating, a TiO₂/Fe₂O₃, SiO₂, TiO₂/Fe₂O₃ coating or a TiO₂, SiO₂, TiO₂ coating.

7. Use according to one of Claims 1 to 3, **characterised in that** component B comprises mica flakes coated with organic and/or inorganic dyes of synthetic or natural origin, spherical SiO₂, coated or uncoated, carbon black, organic coloured pigments and/or inorganic coloured pigments.

8. Use according to one of Claims 1 to 7, **characterised in that** component A and component B are mixed in the ratio 90 : 1 to 1 : 90.

9. Use according to one of Claims 1 to 8, **characterised in that** it additionally comprises a UV stabiliser.

## Revendications

1. Utilisation de mélanges de pigments en dispersion dans des formulations cosmétiques, **caractérisés en ce qu'**ils comprennent au moins deux composants, où le composant A comprend des pigments de BiOCl non revêtus sous la forme d'une dispersion dans de l'huile de ricin, de la laque de nitrocellulose, de l'eau, des huiles polaires et non polaires, des polyols, des solvants hydrophiles et hydrophobes, volatils ou non volatils, et le composant B comprend des pigments nacrés choisis parmi
- des pigments nacrés à base de paillettes de Fe₂O₃, de verre, de TiO₂, de SiO₂, d'Al₂O₃, de polymère ou de graphite,
- des pigments multicouches,
- des pigments à base de Fe₂O₃- de sous-oxyde de TiO₂-, de TiO₂/Fe₂O₃-, de Fe₃O₄-, de FeOOH-, de FeOOH/TiO₂-mica et/ou
des charges et/ou colorants en forme de paillettes, en forme d'aiguilles ou sphériques.

2. Utilisation de mélanges de pigments en dispersion dans des formulations cosmétiques, **caractérisés en ce qu'**ils comprennent au moins deux composants, où le composant A comprend des pigments de BiOCl revêtus sous la forme d'une dispersion dans de l'huile de ricin, de la laque de nitrocellulose, des huiles polaires et non polaires, des polyols, des solvants hydrophiles et hydrophobes, volatils ou non volatils, et le composant B comprend des pigments nacrés, des charges et/ou colorants en forme de paillettes, en forme d'aiguilles ou sphériques.

3. Utilisation selon la revendication 2, **caractérisée en ce que** le composant A comprend des pigments de BiOCl qui sont revêtus de Fe₂O₃, de Rouge Carmin, de Bleu de Prusse ou de Vert d'Oxyde de Chrome.

4. Utilisation selon la revendication 2 ou 3, **caractérisée en ce que** le composant B est un pigment nacré à base de paillettes de mica, de paillettes de Fe₂O₃, de paillettes de verre, de paillettes de TiO₂, de paillettes de SiO₂, de paillettes d'Al₂O₃, de paillettes de polymère ou de paillettes de graphite.

5. Utilisation selon l'une des revendications 1 à 4, **caractérisée en ce que** le pigment nacré est un pigment multicouches.

6. Utilisation selon la revendication 5, **caractérisée en ce que** le composant B comprend un pigment multicouches ayant un revêtement de Fe₂O₃, SiO₂, Fe₂O₃, un revêtement de TiO₂/Fe₂O₃, SiO₂, TiO₂/Fe₂O₃ ou un revêtement de TiO₂, SiO₂, TiO₂.

7. Utilisation selon l'une des revendications 1 à 3, **caractérisée en ce que** le composant B comprend des paillettes de mica revêtues de colorants organiques et/ou inorganiques d'origine synthétique ou naturelle, du SiO₂ sphérique, revêtu ou non revêtu, du noir de charbon, des pigments colorés organiques et/ou des pigments colorés inorganiques.

8. Utilisation selon l'une des revendications 1 à 7, **caractérisée en ce que** le composant A et le composant B sont mélangés selon des rapports allant de 90:1 à 1:90.

9. Utilisation selon l'une des revendications 1 à 8, **caractérisée en ce qu'**elle comprend an outre un stabilisant anti-UV.
